# EUROPÄISCHE PATENTANMELDUNG

(11) **EP 3 020 699 A1**
(43) Veröffentlichungstag der Anmeldung: **18.05.2016**
(21) Anmeldenummer: 14193236.8
(22) Anmeldetag: 14.11.2014
(51) Int. Cl.: C07C 69/54, C09D 147/00, C08F 236/20

(54) **1,3-Propandiol-Derivate**

(71) Anmelder: BASF SE, 67056 Ludwigshafen am Rhein (DE)
(72) Erfinder: Schwalm, Reinhold, 67157 Wachenheim (DE); Charrak, Monika, 67063 Ludwigshafen (DE); Haaf-Kleinhubbert, Christina, 69502 Hemsbach (DE); Thomas, Hans-Josef, 41352 Korschenbroich (DE); Karl, Ulrich, 67269 Grünstadt (DE)
(74) Vertreter: Reinhardt, Jürgen

(57) **Zusammenfassung**

Die Erfindung betrifft 1,3-Propandiol-Derivate der Formel (1) 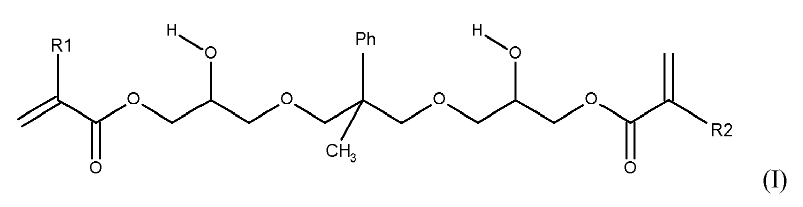 worin die Reste R1 und R2 unabhängig voneinander Wasserstoff oder Methyl bedeuten. Die Verbindungen (I) eignen sich zur Beschichtung der Oberflächen fester Substrate.

## Beschreibung

### Gebiet der Erfindung

Die vorliegende Erfindung betrifft 1,3-Propandiol-Derivate spezieller Struktur, die sich zur Beschichtung der Oberflächen fester Substrate, insbesondere von Kunststoffen, eignen.

### Stand der Technik

Aufgrund ihrer hohen Reaktivität und des niedrigen Preises sind Epoxyacrylate, insbesondere solche auf Basis von Bisphenol-A-diglycidyether, seit langem eine weitverbreitete Bindemittelklasse in strahlungshärtbaren Lacksystemen, insbesondere für die Beschichtung von Holz, Verpackungen und Metallen. In der Regel weisen diese Epoxyacrylate bei Raumtemperatur eine sehr Viskosität auf, was ihre Handhabbarkeit erschwert. Daher müssen diese Verbindungen bei ihrem Einsatz zunächst erhitzt werden, um in einen gerade noch handhabbaren Viskositätsbereich zu kommen. Ein weiterer Nachteil besteht darin, dass Bisphenol-A auf Grund von vermuteten endokrinen Wirkungen bereits teilweise in spezifischen Anwendungen verboten ist und man das Vorhandensein von Restmengen bzw. Freisetzungsprodukten in den Lackschichten nicht ganz ausschließen kann. Daher besteht ein Bedürfnis nach Alternativen von Bisphenol-A-basierten Epoxyacrylaten für strahlungshärtbare Beschichtungen.

WO-A-2014/072216 beschreibt 2-Phenyl-1,3-propandiol-diglycidylether-derivate und darauf basierend Epoxidharze. Auf Seite 4, Zeile 10-11 und Zeilen 25 ff wird 2-Methyl-2-phenyl-1,3-propandiol-diglycidylether genannt. Die Härtung der in WO-A-2014/072216 beschriebenen Substanzen erfolgt auf klassische Weise. Umsetzungsprodukte der in WO-A-2014/072216 genannten 2-Phenyl-1,3-propandiol-diglycidylether-derivate mit Acrylsäure oder Methacrylsäure sind weder offenbart noch nahegelegt.

### Beschreibung der Erfindung

Aufgabe der vorliegenden Erfindung war es, neue Epoxyacrylate zur Verfügung zu stellen, die sich zur Beschichtung der Oberflächen fester Substrate eignen, wobei die Aushärtung der Beschichtung durch Strahlenhärtung, insbesondere mit UV-Licht, erfolgt. Die ausgehärteten Beschichtungen, insbesondere die UV-gehärteten Lacke, sollten sich dabei durch hohe Flexibilität auszeichnen. Insbesondere sollten die zu entwickelnden Substanzen zugleich folgende drei Kriterien erfüllen:
(1) Die Viskosität der Substanzen als solche sollte unterhalb von 20 Pas (Pas = Pascalsekunden) liegen (gemessen mit einem Brookfield-Viskosimeter bei 23°C, gemäß DIN EN ISO 3219/A.3).
(2) Die Pendelhärte der Beschichtungen, die resultieren, wenn die Substanzen auf die Oberflächen fester Substrate aufgebracht und mittels UV-Strahlung gehärtet worden sind, sollte weniger als 100 sec betragen (Pendelhärte nach König, gemessen nach DIN 53157).
(3) Die Erichsentiefung der Beschichtungen, die resultieren, wenn die Substanzen auf die Oberflächen fester Substrate aufgebracht und mittels UV-Strahlung gehärtet worden sind, sollte mehr als 3 mm betragen.

Gegenstand der Erfindung sind zunächst 1,3-Propandiol-Derivate der Formel (I) 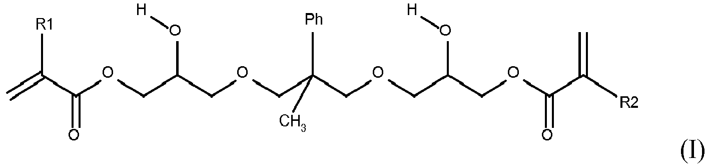 worin die Reste R1 und R2 unabhängig voneinander Wasserstoff oder Methyl bedeuten.

Die Verbindungen (I) können an sich nach allen dem Chemiker bekannten Verfahren hergestellt werden. Vorzugsweise stellt man die Verbindungen (I) wie folgt her: Man setzt Acrylsäure und/oder Methacrylsäure mit 2-Methyl-2-phenyl-1,3-propandiol-diglycidylether (II) 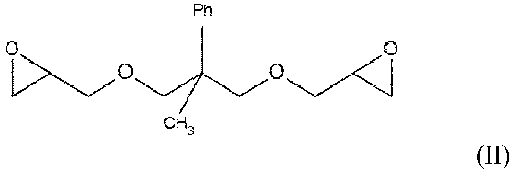 um, wobei die 1,3-Propandiol-Derivate der oben genannten Struktur (I) resultieren.

In diesem Zusammenhang sei festgestellt, dass bei der Umsetzung von (II) mit Acryl- und/oder Methacrylsäure die beiden Oxiranringe von (II) bei der Ringöffnung im Prinzip jeweils in zwei Weisen geöffnet werden können, so dass bei der Ringöffnung ein Gemisch verschiedener Verbindungen resultieren kann. Faktisch dominiert jedoch die Struktur (I). Die in untergeordneten Mengen bei der Ringöffnung von (II) mitentstehenden Species lassen sich durch die nachfolgend angegebenen Formeln (1-a), (1-b) und (1-c) beschreiben, worin die Reste R1 und R2 jeweils unabhängig voneinander Wasserstoff oder Methyl bedeuten.

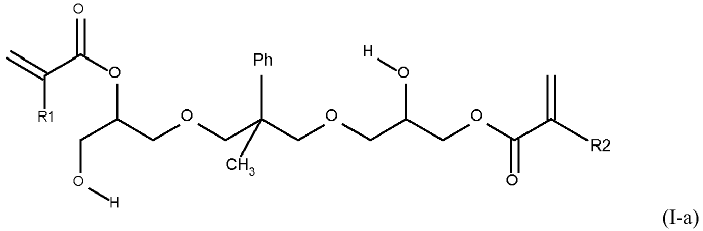

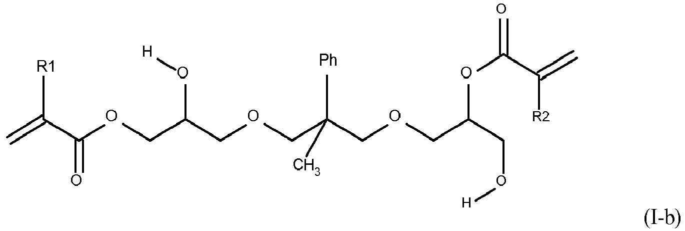

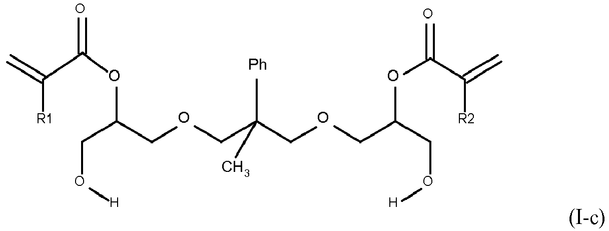

Ein weiterer Erfindungsgegenstand sind Zusammensetzungen enthaltend ein oder mehrere 1,3-Propandiol-Derivate der Formel (I) 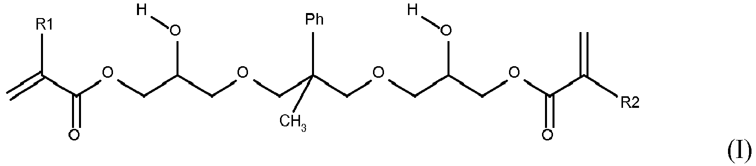 worin die Reste R1 und R2 unabhängig voneinander Wasserstoff oder Methyl bedeuten.

In einer Ausführungsform stellt man solche Zusammensetzungen dadurch her, dass man Di-und/oder Polyole mit überschüssiger Acryl- bzw. Methacrylsäure verestert, und nicht umgesetzte Acryl- bzw. Methacrylsäure anschließend mit 2-Methyl-2-phenyl-1,3-propandioldiglycidylether (II) 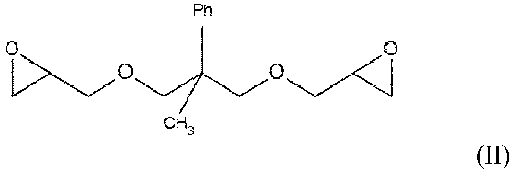 umsetzt, wobei die 1,3-Propandiol-Derivate der oben genannten Struktur (I) resultieren. In dem Produktgemisch liegen dann zum einen die Acryl- bzw. Methacrylsäureester der eingesetzten Di- bzw. Polyole vor, zum anderen die 1,3-Propandiol-Derivate (I).

Ein weiterer Erfindungsgegenstand ist die Verwendung der Verbindungen (I) 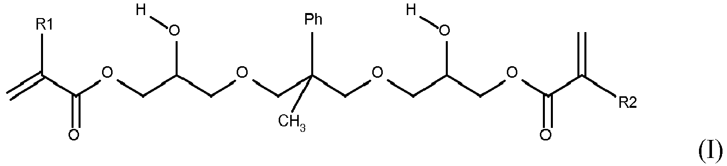 worin die Reste R1 und R2 unabhängig voneinander Wasserstoff oder Methyl bedeuten, als Beschichtungsmassen zur Beschichtung der Oberflächen fester Substrate. Dabei ist die Art des Substrats an sich nicht beschränkt. Beispiele für geeignete Substrate sind beispielsweise Textil, Leder, Metall, Kunststoff, Glas, Holz, Papier oder Pappe.

Ein weiterer Erfindungsgegenstand ist die Verwendung von Zusammensetzungen enthaltend ein oder mehrere 1,3-Propandiol-Derivate der Formel (I) 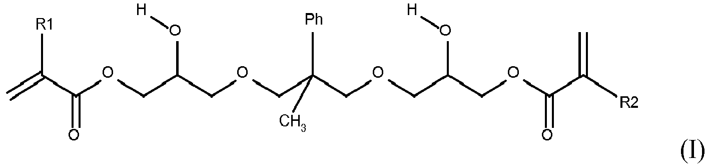 worin die Reste R1 und R2 unabhängig voneinander Wasserstoff oder Methyl bedeuten, als Beschichtungsmassen zur Beschichtung der Oberflächen fester Substrate. Dabei ist die Art des Substrats an sich nicht beschränkt. Beispiele für geeignete Substrate sind beispielsweise Textil, Leder, Metall, Kunststoff, Glas, Holz, Papier oder Pappe.

Der Begriff "Beschichtungsmassen" umfasst jegliche Art von Zusammensetzungen, die auf die Oberfläche eines zu beschichtenden Substrates aufgetragen und anschließend - gegebenenfalls nach vorheriger Trocknung - ausgehärtet wird. Insbesondere schließt der Begriff "Beschichtungsmassen" alle Lackarten ein.

Dabei ist - wie dem Fachmann bekannt - unter "Lack" eine Beschichtungsmasse zu verstehen, die flüssig oder auch pulverförmig sein kann, und die in dünner Schicht dünn auf einen Gegenstand, also das zu beschichtende Substrat, aufgetragen wird und dann ausgehärtet wird. Vergleiche hierzu auch den unten stehenden Abschnitt zum Begriff "Beschichten".

Die erfindungsgemäßen Beschichtungsmassen können neben den Verbindungen (I) zusätzlich weitere lacktypische Additive enthalten, beispielsweise Antioxidantien, Stabilisatoren, Aktivatoren (Beschleuniger), Füllmittel, Pigmente, Farbstoffe, antistatische Agentien, Flammschutzmittel, Verdicker, thixotrope Agentien, oberflächenaktive Agentien, Viskositätsmodifikatoren, Plastifizierer oder Chelatbildner verwendet werden. Weiterhin können die erfindungsgemäßen Beschichtungsmassen neben den Verbindungen (I) auch weitere strahlungshärtbare Komponenten enthalten, die nicht von der Formel (I) umfasst sind.

Als Verdicker kommen neben radikalisch (co)polymerisierten (Co)Polymerisaten, übliche organische und anorganische Verdicker wie Hydroxymethylcellulose oder Bentonit in Betracht.

Als Chelatbildner können z.B. Ethylendiaminessigsäure und deren Salze sowie β-Diketone verwendet werden.

Geeignete Füllstoffe umfassen Silikate, z. B. durch Hydrolyse von Siliciumtetrachlorid erhältliche Silikate wie Aerosil® der Fa. Degussa, Kieselerde, Talkum, Aluminiumsilikate, Magnesiumsilikate, Calciumcarbonate etc.

Geeignete Stabilisatoren umfassen typische UV-Absorber wie Oxanilide, Triazine und Benzotriazol (letztere erhältlich als Tinuvin® -Marken der Ciba-Spezialitätenchemie) und Benzophenone. Diese können allein oder zusammen mit geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetra-methyl-4-piperi-dyl)sebacinat, eingesetzt werden. Stabilisatoren werden üblicherweise in Mengen von 0,1 bis 5,0 Gew.-%, bezogen auf die in der Zubereitung enthaltenen festen Komponenten, eingesetzt.

Pigmente können ebenfalls in den Beschichtungsmassen enthalten sein. Pigmente sind gemäß CD Römpp Chemie Lexikon - Version 1.0, Stuttgart/New York: Georg Thieme Verlag 1995 unter Verweis auf DIN 55943 partikelförmige "im Anwendungsmedium praktisch unlösliche, anorganische oder organische, bunte oder unbunte Farbmittel". Praktisch unlöslich bedeutet dabei eine Löslichkeit bei 25 °C unter 1 g / 1000 g Anwendungsmedium, bevorzugt unter 0,5, besonders bevorzugt unter 0,25, ganz besonders bevorzugt unter 0,1 und insbesondere unter 0,05 g / 1000 g Anwendungsmedium.

Wird ein Pigment eingesetzt, so ist darauf zu achten, dass entweder die Härtung mit Elektronenstrahlen durchgeführt wird oder dass ein Photoinitiator verwendet wird, der trotz der Pigmentierung durch die eingestrahlte Strahlung aktiviert werden kann, beispielsweise indem der Photoinitiator eine signifikante Absorbanz in einem Wellenlängenbereich aufweist, in dem das Pigment für die eingestrahlte Strahlung ausreichend durchlässig ist. Es stellt eine bevorzugte Ausführungsform der vorliegenden Erfindung dar, kein Pigment zu verwenden und die Beschichtungsmasse in Klarlacken einzusetzen.

Beispiele für Pigmente umfassen beliebige Systeme von Absorptions- und/oder Effektpigmenten, bevorzugt Absorptionspigmente. Anzahl und Auswahl der Pigmentkomponenten sind dabei keinerlei Beschränkungen unterworfen. Sie können den jeweiligen Erfordernissen, beispielsweise dem gewünschten Farbeindruck, beliebig angepaßt werden.

Unter Effektpigmenten sind alle Pigmente zu verstehen, die einen plättchenförmigen Aufbau zeigen und einer Oberflächenbeschichtung spezielle dekorative Farbeffekte verleihen. Bei den Effektpigmenten handelt es sich beispielsweise um alle in der Fahrzeug- und Industrielackierung üblicherweise einsetzbaren effektgebenden Pigmente. Beispiele für derartige Effektpigmente sind reine Metallpigmente; wie z.B. Aluminium-, Eisen- oder Kupferpigmente; Interferenzpigmente, wie z.B. titandioxidbeschichteter Glimmer, eisenoxidbeschichteter Glimmer, mischoxidbeschichteter Glimmer (z.B. mit Titandioxid und Fe₂O₃ oder Titandioxid und Cr₂O₃), metalloxidbeschichtetes Aluminium, oder Flüssigkristallpigmente.

Bei den farbgebenden Absorptionspigmenten handelt es sich beispielsweise um übliche in der Lackindustrie einsetzbare organische oder anorganische Absorptionspigmente. Beispiele für organische Absorptionspigmente sind Azopigmente, Phthalocyanin-, Chinacridon- und Pyrrolopyrrolpigmente. Beispiele für anorganische Absorptionspigmente sind Eisenoxidpigmente, Titandioxid und Ruß.

Sofern die Aushärtung der Beschichtungsmassen nicht mit Elektronenstrahlen, sondern mittels UV-Strahlung erfolgt, ist vorzugsweise wenigstens ein Photoinitiator enthalten, der die Polymerisation ethylenisch ungesättigter Doppelbindungen (C=C-Doppelbindungen) initiieren kann.

Ganz allgemein können alle dem Fachmann einschlägig bekannten Photoinitiatoren ein gesetzt werden, wie sie beispielsweise in einschlägigen Fachpublikationen und Monographien beschrieben sind.

In Betracht kommen zum Beispiel:
- Mono- oder Bisacylphosphinoxide, etwa 2,4,6-Trimethylbenzoyldiphenylphosphinoxid (Lucirin® TPO der BASF SE), Ethyl-2,4,6-trimethylbenzoylphenylphosphinat (Lucirin® TPO L der BASF SE), Bis-(2,4,6-trimethylbenzoyl)-phenylphosphinoxid (Irgacure® 819 der Firma Ciba Spezialitätenchemie),
- Benzophenone, Hydroxyacetophenone, Phenylglyoxylsäure und ihre Derivate oder Gemische dieser Photoinitiatoren. Als Beispiele seien genannt: Benzophenon, Acetophenon, Acetonaphthochinon, Methylethylketon, Valerophenon, Hexanophenon, α-Phenylbutyrophenon, p-Morpholinopropiophenon, Dibenzosuberon, 4-Morpholinobenzophenon, 4-Morpholinodeoxybenzoin, p-Diacetylbenzol, 4-Aminobenzophenon, 4'-Methoxyacetophenon, β-Methylanthrachinon, tert-Butylanthrachinon, Anthrachinoncarbonysäureester, Benzaldehyd, α-Tetralon, 9-Acetylphenanthren, 2-Acetylphenanthren, 10-Thioxanthenon, 3-Acetylphenanthren, 3-Acetylindol, 9-Fluorenon, 1-Indanon, 1,3,4-Triacetylbenzol, Thioxanthen-9-on, Xanthen-9-on, 2,4-Dimethylthioxanthon, 2,4-Diethylthioxanthon, 2,4-Di-iso-propylthioxanthon, 2,4-Dichlorthioxanthon, Benzoin, Benzoin-iso-butylether, Chloroxanthenon, Benzointetrahydropyranylether, Benzoin-methylether, Benzoin-ethylether, Benzoin-butylether, Benzoin-iso-propylether, 7-H-Benzoin-methylether, Benz[de]anthracen-7-on, 1-Naphthaldehyd, 4,4'-Bis(dimethylamino)benzophenon, 4-Phenylbenzophenon, 4-Chlorbenzophenon, Michlers Keton, 1-Acetonaphthon, 2-Acetonaphthon, 1-Benzoylcyclohexan-1-ol, 2-Hydroxy-2,2-dimethylacetophenon, 2,2-Dimethoxy-2-phenylacetophenon, 2,2-Diethoxy-2-phenylacetophenon, 1,1-Dichloracetophenon, 1-Hydroxyacetophenon, Acetophenondimethylketal, o-Methoxybenzophenon, Triphenylphosphin, Tri-o-Tolylphosphin, Benz[a]anthracen-7,12-dion, 2,2-Diethoxyacetophenon, Benzilketale, wie Benzildimethylketal, 2-Methyl-1-[4-(methylthio)phenyl]-2-morpholinopropan-1-on, Anthrachinone wie 2-Methylanthrachinon, 2-Ethylanthrachinon, 2-tert-Butylanthrachinon, 1-Chloranthrachinon, 2-Amylanthrachinon und 2,3-Butandion.

Geeignet sind auch nicht- oder wenig vergilbende Photoinitiatoren vom Phenylglyoxalsäureester-Typ.

Es können auch Gemische verschiedener Photoinitiatoren eingesetzt werden. Typische Gemische umfassen beispielsweise 2-Hydroxy-2-Methyl-1-phenyl-propan-2-on und 1-Hydroxycyclohexyl-phenylketon, Bis(2,6-dimethoxybenzoyl)-2,4,4-trimethylpentylphosphinoxid und 2-Hydroxy-2-methyl-1-phenyl-propan-1-on, Benzophenon und 1-Hydroxy-cyclohexyl-phenylketon, Bis(2,6-dimethoxybenzoyl)-2,4,4-trimethylpentylphosphinoxid und 1-Hydroxycyclohexyl-phenylketon, 2,4,6-Trimethylbenzoyldiphenylphosphinoxid und 2-Hydroxy-2-methyl-1-phenyl-propan-1-on, 2,4,6-Trimethylbenzophenon und 4-Methylbenzophenon oder 2,4,6-Trimethylbenzophenon und 4-Methylbenzophenon und 2,4,6-Trimethylbenzoyldiphenylphosphinoxid.

Bevorzugte Photoinitiatoren sind:
- 2,4,6-Trimethylbenzoyldiphenylphosphinoxid,
- Ethyl-2,4,6-trimethylbenzoylphenylphosphinat,
- Bis-(2,4,6-trimethylbenzoyl)-phenylphosphinoxid,
- Benzophenon,
- 1-Benzoylcyclohexan-1-ol,
- 2-Hydroxy-2,2-dimethylacetophenon und
- 2,2-Dimethoxy-2-phenylacetophenon.

Die Beschichtungsmassen enthalten die Photoinitiatoren vorzugsweise in einer Menge von 0,05 bis 10 Gew.-%, besonders bevorzugt 0,1 bis 8 Gew.-%, insbesondere 0,2 bis 5 Gew.-%, bezogen auf die Gesamtmenge der in den Beschichtungsmassen vorhandenen härtbaren Komponenten.

Die Beschichtung der Oberflächen fester Substrate mit den erfindungsgemäß einzusetzenden Tetrahydrofuranderivaten (I) erfolgt nach üblichen, dem Fachmann bekannten Verfahren, wobei man das gewünschte Tetrahydrofuranderivat (I) bzw. eine Beschichtungsmasse, die ein oder mehrere Verbindungen (I) enthält in der gewünschten Stärke auf das Substrat aufbringt und zumindest teilweise strahlungshärtet. Dabei ist eine vollständige Strahlenhärtung bevorzugt. Dieser Vorgang kann gewünschtenfalls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Co-Extrudieren, bevorzugt durch Spritzen und Walzen erfolgen. Als Spritzverfahren können z.B. Luftdruck-, Airless- oder Elektrostatik-Spritzverfahren Anwendung finden.

Wie dem Fachmann bekannt ist unter Strahlenhärtung die radikalische Polymerisation von polymerisierbaren Verbindungen zu verstehen, die induziert wird durch elektromagnetische und/oder korpuskularen Strahlung. Der Einsatz von UV-Licht oder Elektronenstrahlung (Elektronenstrahlung; 150 bis 300 keV) ist bevorzugt. Insbesondere ist UV-Licht im Wellenlängenbereich von 200 bis 500 nm und insbesondere von 250 bis 400 nm bevorzugt.

Die Beschichtungsstärke wird vorzugsweise so eingestellt, dass die Trockenfilmdicke im Bereich von 30 bis 200 µm, und vorzugsweise im Bereich von 50-150 µm liegt. Wie dem Fachmann bekannt, versteht man unter Trockenschichtdicke die Schichtdicke einer getrockneten bzw. ausgehärteten Beschichtung. Der Begriff des Trocknens schließt dabei ein, dass in einer Beschichtungsmasse vorhandene Lösungsmittel, z.B. Wasser oder organische Lösungsmittel, verdunstet sind. Der Begriff des Aushärtens schließt dabei ein, dass eine Vernetzung der Beschichtungsmasse erfolgt. Es sei eigens betont, dass der Begriff der Trockenschichtdicke hier rein phänomenologisch zu verstehen ist als diejenige Schichtdicke, die eine getrocknete und/oder ausgehärteten Beschichtung aufweist.

Die Strahlenhärtung kann gewünschtenfalls bei höheren Temperaturen erfolgen. Bevorzugt ist dabei eine Temperatur oberhalb der T_{g}-Wertes des strahlungshärtbaren Bindemittels (T_{g}-Wert = Glasübergangstemperatur).

Die Strahlenhärtung kann unter sauerstoffhaltiger Atmosphäre oder unter Inertgas erfolgen, wobei letzteres bevorzugt ist.

Neben einer Strahlenhärtung können noch weitere Härtungsmechanismen involviert sein, beispielsweise thermische-, Feuchtigkeits-, chemische und/oder oxidative Härtung.

Gegebenenfalls kann, wenn mehrere Schichten des Beschichtungsmittels übereinander aufgetragen werden, nach jedem Beschichtungsvorgang eine Trocknung und/oder Strahlenhärtung erfolgen.

Als Strahlungsquellen für die Strahlenhärtung geeignet sind z.B. Quecksilber-Niederdruckstrahler, -Mitteldruckstrahler mit Hochdruckstrahler sowie Leuchtstoffröhren, Impulsstrahler, Metallhalogenidstrahler, Laser, gepulste Lampen (Blitzlicht), Halogenlampen Elektronenblitzeinrichtungen, wodurch eine Strahlenhärtung ohne Photoinitiator möglich ist, oder Excimerstrahler.

Es können auch mehrere Strahlungsquellen für die Strahlenhärtung eingesetzt werden, z.B. zwei bis vier. Diese können gewünschtenfalls auch in jeweils unterschiedlichen Wellenlängebereichen strahlen.

Die Bestrahlung kann gegebenenfalls auch unter Ausschluss von Sauerstoff, z. B. unter Inertgas-Atmosphäre, durchgeführt werden. Als Inertgase eignen sich vorzugsweise Stickstoff, Edelgase, Kohlendioxid, oder Verbrennungsgase.

Ein weiterer Erfindungsgegenstand ist gemäß dem oben Ausgeführten ein Verfahren zum Beschichten der Oberflächen fester Substrate, wobei man 1,3-Propandiol-Derivate der Formel (I) 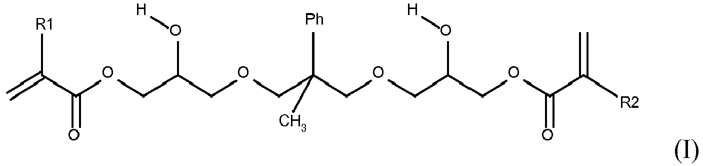 worin die Reste R1 und R2 unabhängig voneinander Wasserstoff oder Methyl bedeuten, oder Zusammensetzungen, die ein oder mehrere Verbindungen (I) enthalten, auf die Oberfläche eines festen Substrates aufbringt und anschließend eine Strahlenhärtung durchführt, insbesondere mittels UV-Licht.

Unter "Beschichten", auch "coating" genannt, sind dabei Verfahren zu verstehen, die dem Aufbringen einer festhaftenden Schicht auf die Oberfläche eines Werkstückes - dem Substrat - dienen. Die aufgetragene Schicht wird als Beschichtung bezeichnet. Die üblichen Beschichtungsverfahren unterscheiden sich durch die Art der Aufbringung der Beschichtungsmassen in chemische, mechanische, thermische und thermomechanische Verfahren. Im Rahmen der vorliegenden Erfindung ist die UV-Härtung bevorzugt, die eine chemische Vernetzung der in den Beschichtungsmassen enthaltenen Verbindungen (I) induziert.

### Mess- und Prüfmethoden

**Viskosität:** Die Viskosität der Substanzen als solche wurden gemessen mit einem Brookfield-Viskosimeter bei 23 °C, gemäß DIN EN ISO 3219/A.3.

**Pendeldämpfung (PD):** Die Pendeldämpfung (oft auch als Pendelhärte bezeichnet) von Beschichtungen, die resultierten, wenn die zu prüfenden Substanzen auf die Oberflächen fester Substrate aufgebracht und mittels UV-Strahlung gehärtet worden waren, die sogenannten Pendelhärte nach König, wurden gemessen nach DIN 53157. Bei dieser Methode wird die Pendeldämpfung in Sekunden angegeben.

**Erichsentiefung (Ew):** Die Erichsentiefung ist ein Maß für die Elastizität von Beschichtungen. Die Erichsentiefung von Beschichtungen, die resultierten, wenn die zu prüfenden Substanzen auf die Oberflächen fester Substrate aufgebracht und mittels UV-Strahlung gehärtet worden waren, wurden gemessen gemäß DIN ISO 1520. Die Erichsentiefung wird in [mm] angegeben.

**Iodfarbzahl:** Die Messung der Iodfarbzahl erfolgte mit dem Gerät Lange Lico 400 gemäß DIN 6162

### Eingesetzte Substanzen

**MPPD-DG:** 2-Methyl-2-phenyl-1,3-propandiol-diglycidylether. Die chemische Struktur dieser Substanz ist diejenige der obigen Formel (II).

**MPPD-DGDA:** MPPD-DGDA wurde durch Umsetzung von MPPD-DG mit Acrylsäure erhalten. Dabei entsteht überwiegend 2-Methyl-2-phenyl-1,3-propandiol-diglycidylether-diacrylat, was der obigen Formel (I), wobei beide Reste R1 und R2 Wasserstoff bedeuten, entspricht. Spezies der oben genannten Strukturen (1-a), (1-b) und (1-c) sind lediglich in sehr geringen Mengen mitenthalten.

**PEA-Acrylsäure-Zusammensetzung:** In einer 2-Liter Apparatur wurde eine Mischung von 694,6 g ethoxyliertes Trimethylolpropan (mit einer OH-Zahl von 607 mg KOH/g), 190 g Adipinsäure, 327,3 g Methylcyclohexan, 424,5 g Acrylsäure, 1,3g Hypophosphorige Säure und 6,5 g konz. Schwefelsäure vorgelegt. Zu dieser Mischung wurden als Stabilisatoren 1,3g 2,6 tert-butyl-p-Kresol (=Kerobit), 3,9 g Methylhydrochinon und 0,04g Phenothiazin zudosiert. Das Gesamtsystem wurde auf eine Temperatur von 109 °C erhitzt und Wasser über einen Zeitraum von 6,5 Stunden ausgekreist. Anschließend wurden das Methylcyclohexan und überschüssige Acrylsäure bis zu einer Säurezahl (SZ) von 43,6 mg KOH/g Substanz im Vakuum entfernt. Die erhaltene Zusammensetzung stellt eine Zusammensetzung dar, die Polyesteracrylate enthält, jedoch auch noch freie Acrylsäure.

**BADGE-DA:** Bisphenol-A-diglycidylether-diacrylat (CAS Nummer = 4687-94-9), Handelsprodukt "Miramer PE 210" der Fa. Miwon. Das Produkt wies bei 60 °C eine Viskosität von 500 Pas auf.

### Beispiele

In den Anwendungsbeispielen 2 und 4 wurde zur Aushärtung der Beschichtungsmassen eine IST-UV-Anlage eingesetzt (Anlagentyp: M-40-2x1-R-TR-SLC-SO-Inert; Lampe 1: IST-UV-Lampe M400 U2HC; Lampe 2: IST-UV-Lampe M400 U2H).

### Beispiel 1

### Herstellung von MPPD-DGDA:

85 g MPPD-DG (mit einem Epoxydäquivalent von 187 g/eg), 33 g Acrylsäure, 0,1 g Phenothiazin, 3 g Hydrochinon-monomethylether und 3,5 g Triphenylphosphin wurden bei Raumtemperatur gemischt und 10 Stunden bei 100°C reagiert. Nachdem die Säurezahl auf 5mg KOH/g abgefallen war, wurde abgekühlt. Es entstand ein gelblich klares Produkt.

Das H-NMR Spektrum zeigte an, dass die Epoxidgruppe komplett abreagiert hatte (H-Signale bei 5,85 ppm, 6,15 ppm und 6,45 ppm, typisch für acrylische Doppelbindungen).

Die Viskosität betrug 14 Pas.

### Beispiel 2

### Bestimmung der Filmeigenschaften des Produktes gemäß Beispiel 1

10 g des in Beispiel 1 hergestellten Diacrylates MPPD-DGDA wurden mit 0,4 g des Photoinitiators Irgacure 500 gemischt. Die so erhaltene Lackformulierung wurde mit einem 100 µm Kastenrakel auf Bonderblech appliziert und mit 1350 mJ/cm2 auf einer IST-UV Bandanlage unter Luft belichtet.

Folgende Filmwerte wurden bestimmt:

| | |
|---|---|
| Pendeldämpfung: | 28 s |
| Erichsentiefung: | 5,6 mm |

### Beispiel 3

### Herstellung einer Zusammensetzung enthaltend Polyesteracrylate und MPPD-DGDA

Zu einer Mischung PEA-Acrylsäure-Zusammensetzung, deren Herstellung oben beschrieben ist, und die 185 g Polyesteracrylate und 56g Acrylsäure enthielt, wurden 4,3 g Triphenylphosphin und 36,33g MPPD-DG gegeben. Es wurde bei 20 °C gemischt und 8,5 Stunden bei 108°C reagiert. Nachdem die Säurezahl auf 4mg KOH/g abgefallen war, wurde abgekühlt. Es wurde ein gelblich klares Produkt erhalten.

| | |
|---|---|
| Die Viskosität betrug | 1,2 Pas. |
| Die Jodfarbzahl lag bei | 3,4. |

### Beispiel 4

### Bestimmung der Filmeigenschaften der Zusammensetzung gemäß Beispiel 3

10 g der in Beispiel 3 hergestellten Zusammensetzung wurden mit 0,4 g des Photoinitiators Irgacure 500 gemischt. Die so erhaltene Lackformulierung wurde mit einem 150 µm Kastenrakel auf Bonderblech appliziert und mit 1900 mJ/cm² auf einer IST-UV Bandanlage unter Stickstoff belichtet.

Folgende Filmwerte wurden bestimmt:

| | |
|---|---|
| Pendeldämpfung: | 49 s |
| Erichsentiefung: | 3,6 mm |

### Beispiel 5 (zum Vergleich)

### Bestimmung der Filmeigenschaften von BADGE-DA, Miramer PE 210

10 Teile von BADGE-DA, wurden mit 0,4 Teilen des Photoinitiators Irgacure 500 gemischt. Die so erhaltene Lackformulierung wurde aufgrund der hohen Viskosität von BADGE-DA mit 50 °C mit einem 150 µm Kastenrakel auf Bonderblech appliziert und mit 1900 MJ/cm² auf einer IST-UV Bandanlage unter Stickstoff belichtet.

Folgende Filmwerte wurden bestimmt:

| | |
|---|---|
| Pendeldämpfung: | 186 s |
| Erichsentiefung: | 2,1 mm |

## Patentansprüche

1. 1,3-Propandiol-Derivate der Formel (I) 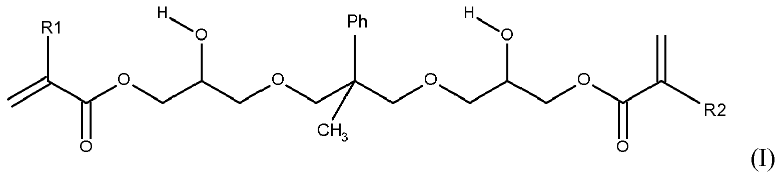 worin die Reste R1 und R2 unabhängig voneinander Wasserstoff oder Methyl bedeuten.

2. 1,3-Propandiol -Derivate nach Anspruch 1, mit der Maßgabe, dass beide Reste R1 und R2 Wasserstoff bedeuten.

3. Zusammensetzungen enthaltend ein oder mehrere 1,3-Propandiol-Derivate der Formel (I) 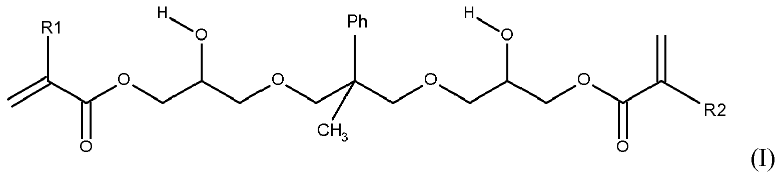 worin die Reste R1 und R2 unabhängig voneinander Wasserstoff oder Methyl bedeuten.

4. Verwendung von 1,3-Propandiol-Derivaten der Formel (I) 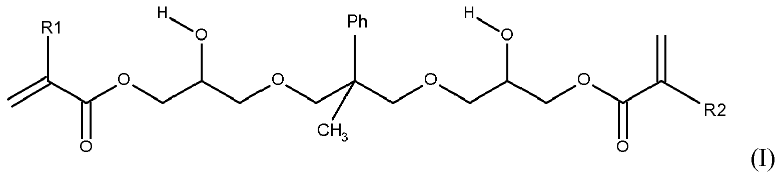 worin die Reste R1 und R2 unabhängig voneinander Wasserstoff oder Methyl bedeuten als Beschichtungsmassen zur Beschichtung der Oberflächen fester Substrate.

5. Verwendung von Zusammensetzungen enthaltend ein oder mehrere 1,3-Propandiol-Derivate der Formel (I) 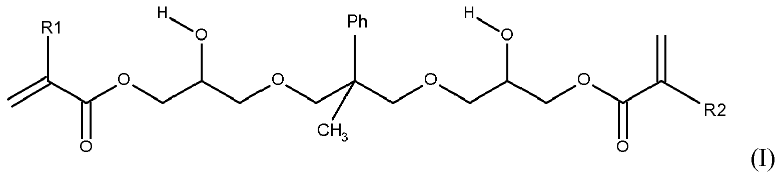 worin die Reste R1 und R2 unabhängig voneinander Wasserstoff oder Methyl bedeuten, als Beschichtungsmassen zur Beschichtung der Oberflächen fester Substrate.

6. Verfahren zum Beschichten der Oberflächen fester Substrate, wobei man 1,3-Propandiol-Derivate (I) 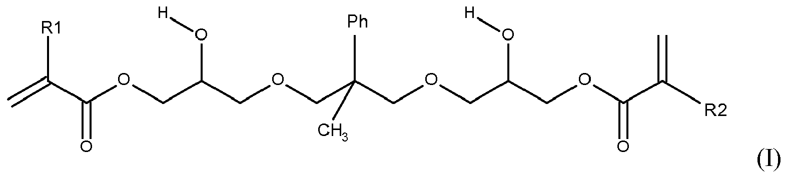 worin die Reste R1 und R2 unabhängig voneinander Wasserstoff oder Methyl bedeuten, oder Beschichtungsmassen, die ein oder mehrere Verbindungen (I) enthalten, auf die Oberfläche eines festen Substrates aufbringt und anschließend eine Strahlenhärtung durchführt.

7. Verfahren nach Anspruch 6, wobei es sich bei der Strahlenhärtung um eine Härtung mit UV-Licht der Wellenlänge im Bereich von 200 bis 500 nm handelt.

8. Verfahren nach Anspruch 6, wobei es sich bei den festen Substraten um Kunststoffe und bei der Strahlenhärtung um eine Härtung mit UV-Licht der Wellenlänge im Bereich von 250 bis 400nm handelt.
